# EUROPEAN PATENT SPECIFICATION

(11) **EP 0 132 242 B1**
(45) Date of publication and mention of the grant of the patent: **27.10.1993**
(21) Application number: 84850212.6
(22) Date of filing: 04.07.1984
(51) Int. Cl.: A61K 31/70, C12Q 1/04

(54) **A compound and a composition for therapeutic or diagnostic use**
Verbindung und Zusammensetzung zur therapeutischen oder diagnostischen Anwendung
Un composé et une composition pour application thérapeutique ou diagnostique

(30) Priority: 15.07.1983 SE 8304007
(43) Date of publication of application: 23.01.1985
(73) Proprietor: SYMBICOM AB, S-901 24 UMEA (SE)
(72) Inventor: Karlsson, Karl-Anders, S-411 43 Goteborg (SE); Lindberg, Alf Anton, S-182 75 Stocksund (SE)
(74) Representative: Burman, Tore

(56) References cited:
- EP-A- 0 089 940
- WO-A-81/02520
- WO-A-81/03175
- US-A- 3 891 508
- EUR. J. BIOCHEM., vol. 76, 1977; B.A.DMITRIEV et al., pp. 433-440.
- J. BIOL. CHEM., vol. 262, no. 4, 05 February 1987; A.LINDBERG et al., pp. 1779-1785.
- CHEMICAL ABSTRACTS, vol. 87, no. 11, 1 September 1977, Columbus, OH (US); H.ARITA et al., p. 333, no. 81790d.
- CHEMICAL ABSTRACTS, vol. 96, no. 17, 26 April 1982, Columbus, OH (US); J.AANGSTROEM et al., p. 350, no. 138362h; and J.AANGSTROEM et al., p. 351, no. 138364k.
- J. BIOL. CHEM., vol. 255, no. 1, January 1980, US; S.OLSNES et al., pp. 284-289.
- CHEMICAL ABSTRACTS, vol. 93, no. 21, 24 November 1980, Columbus, OH (US); M.K.GENTRY et al., p. 145, no. 198591e.

## Description

The present invention relates to compounds and compositions which are useful for therapeutic treatment of disorders caused by the toxine of Shigella Dysenteriae as well as prophylaxis and diagnosis in connection herewith.

Necrosis of the epithelium of the colon is part of the pathogenesis of bacillary dysenteria after infection with Shigella Dysenteriae (see Davis, B.D., Dulbecco, R., Eisen, H.N., and Ginsberg, N.S.(eds) Microbiology Harper and Row, Publishers, Philadelphia, Third Edition, 1980). The necrosis is due to an invasion of the bacterium into the epithelium to produce therein a toxine which appears to inhibit protein synthesis (see Brown, J.E. Rothman, S.W., and Doctor, B.P. (1980) Infect. Immun. 29, 98-107). The purified toxine has a molecular weight of about 70 000 (see Brown, J.E., Griffin, D.E.,Rothman, S.W., and Doctor, B.P. (1982) Infect. Immun. 36, 996-1005) and is composed of one heavy and four to five light subunits similar to the case for cholera toxine (see Olsnes, S., and Eiklid, K. (1980) J.Biol.Chem. 255, 284-289). The toxine has recently been purified on a milligram scale (see Brown, J.E., Griffin, D.E., Rothman, S.W., and Doctor, B.P. (1982) Infect. Immun. 36. 996-1005), which has enabled more detailed studies of its mechanism of action.

Carbohydrate structures are known which function as receptors for different types of bacteria, such as bacteria causing infections in mammals. PCT-application WO 81/02520 constitutes a disclosure concerning such prior art and is related to a specific structural element: 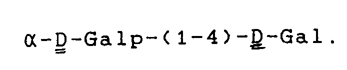
It is clear from the specific disclosure of said application that said structural element is always placed in terminal position in the overall structure. Such carbohydrate structure thus has the ability of replacing the normal receptor function in vivo in relation to certain pathogenic bacteria constituting a basis for using the structure therapeutically or diagnostically.

The present invention has for its purpose of providing carbohydrate structures which can be used therapeutically for the treatment of disorders due to infection with Shigella Dysenteriae and which can also be used for diagnosing the toxine formed by the mentioned bacterium.

In connection with extensive research and experimentation it has now been found that the active receptor substance vis a vis the toxine of Shigella Dysenteriae has the formula (I): 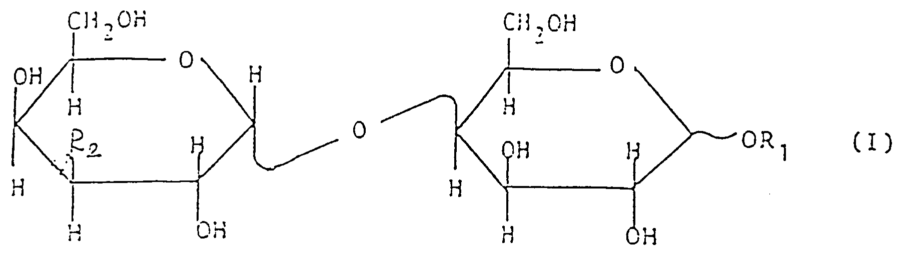
wherein R₁ is hydrogen or an organic residue, provided it does not negatively affect the conditions in connection with the use of the invention and R₂ is
β-D-Galp-(1-3)-β-D-GalNAcp-(1-3)-,
α-D-Galp-(1-3)-, or
β-D-GalNAcp-(1-3)-.

In the substance of formula I above it is preferred that OR₁ is in β-configuration.

Examples of active substances within formula I above are the following:
β- 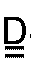 -Galp-(1-3)-β- 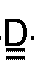 -GalNAcp-(1-3)-α- 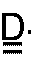 -Galp-(1-4)-β- 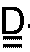 -Galp-(1-4)-β- 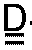 -Glcp-(1-0)-ceramide;
α- 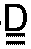 -Galp-(1-3)-α- 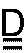 -Galp-(1-4)-β- 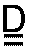 -Galp-(1-4)-β- 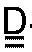 -Glcp-(1-0)-ceramide; and
β- 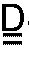 -GalNAcp-(1-3)-α- 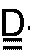 -Galp-(1-4)-β- 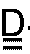 -Galp-(1-4)- 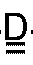 -Glucitol.

The expression "lower" used in this disclosure refers to a group containing 1-6 carbon atoms, particularly 1-4 carbon atoms and especially 1 or 2 carbon atoms.

R₁ may be a carbohydrate residue, but it is also preferred that R₁ is lower alkyl, i.e. having 1 to 6 carbon atoms.

The active substance I of the present invention may be used as such or in combination with a pharmaceutically acceptable carrier.

The active substances according to the present invention can be formulated for use in human or veterinary medicine for therapeutic, prophylactic or diagnostic uses. In clinical practice the active constituents are normally administered orally or rectally or by injection in the form of a pharmaceutical preparation containing the active constituents in combination with a pharmaceutically acceptable carrier, which may be solid, semisolid or liquid, or as a capsule, and such compositions constitute a further aspect of the invention. The compounds may also be used as such without carrier and in a form of an aqueous solution for injection. As examples of pharmaceutical preparations there may be mentioned tablets, drops, solutions and suppositories. The active substance usually constitutes from 0.05 to 99% by weight of the preparation, for example from 0.1 to 50% for preparations intended for oral administration.

To manufacture pharmaceutical preparations in the form of dose units for oral application containing a compound according to the invention the active constituents can be admixed with a solid, pulverulent or other carrier, for example lactose, saccharose, sorbitol, mannitol, starch, such as potatoe starch, corn starch, amylopectin , a cellulose derivative or gelatin and may also include lubricants, such as magnesium or calcium stearate, or polyethylene glycol waxes compressed to form tablets or cause for dragées.

By using several layers of the active drug separated by slowly dissolving layers tablets of delayed release are obtained.

Liquid preparations for oral application can be in the form of elixires, syrups or suspensions, for example solutions containing from 0.1 to 20% by weight of active substance, sugar and a mixture of ethanol, water, glycerol, propylene glycol and optionally other additives of a conventional character.

The dose by which the active constituents are administered may vary within wide limits and depend on different factors, such as the severity of the disorder, the age and the weight of the patient and can be individually adjusted. As a conceivable range for the quantity of active constituents that may be administered per day there may be mentioned from 0.1 to 2000 mg or from 1 mg to 2000 mg.

In regard to the meaning of substituent R₁ of the formula I above this may be of any type as long as it does not negatively affect the conditions in connection with the use of the invention. Thus, R₁ may be hydrogen, lower alkyl or lower acyl but R₁ may also constitute the residue of a natural or synthetic glycoconjugate, the compound according to the invention thus being a glycoconjugate, for example a glycolipid.

Moreover, R₁ in formula I may be a macromolecular carrier, optionally including a coupling arm. Such carrier can be a synthetically or naturally occurring polypeptide, polysaccharide or other type of polymer or particle.

The invention will in the following be further described in connection with non-limiting examples.

In connection with the creation of the invention a new analysis method has been devised which can be defined as a form of chromatographic binding assay. The principle is the following.

A thin layer chromatogram is prepared from pure defined receptor structures and/or receptor structures in a partly unknown mixture extracted from tissue of interest. The thin layer chromatogram is prepared on a surface of silica gel and is treated after the application for avoiding unspecific binding of the substance of interest with a silica gel. The toxine which has been radioactively labelled with J¹²⁵ is then transferred to the treated chromatogram. After washing away excess substance there is shown by autoradiography how it has been bound to the individual separated receptor structures. In this manner there is obtained information concerning which structures are active receptors in relation to the applied toxine and which structures do not not show binding capacity.

Far going description of the binding specificity may hereby be obtained by comparision of closely related structures. The identity for a receptor (binding substance) is suitably obtained by comparison with structurally known reference substances. The receptor structures utilized in this disclosure are known substances.

### EXAMPLES

The experiment was carried out with a thin layer chromatogram on a surface of silica gel prepared as indicated above. The thin layer chromatogram was detected with anisaldehyde, and autoradiogram was performed after binding with Shigella-toxine labeled with the radioactive iodoisotope J¹²⁵.

The results of the toxine binding studies while using a number of carbohydrate structures are given below in the table, a plus indicating active binding capacity, and a minus indicating lack of binding capacity in relation to the toxine. The table also gives the specificity concerning the binding capacity of the structures according to this invention in that already small deviations in the structure from that defined by formula I results in absence of binding.

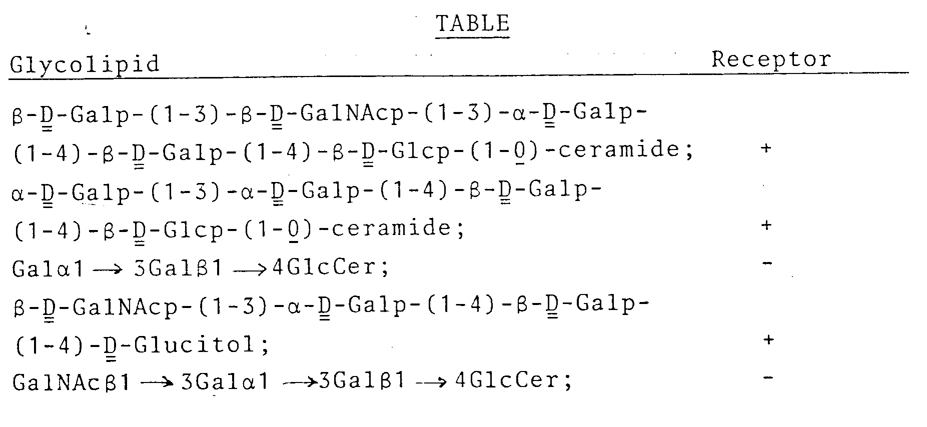

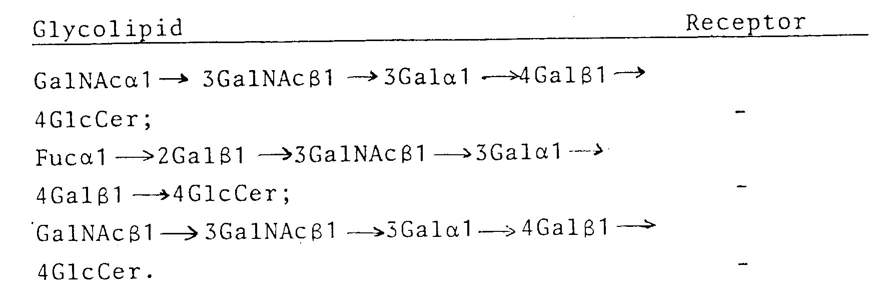

## Claims

1. A composition for therapeutic or diagnostic use due to its ability to bind the toxine of Shigella Dysenteriae, characterized by containing or consisting of, as an active constituent, a compound of the formula (I): 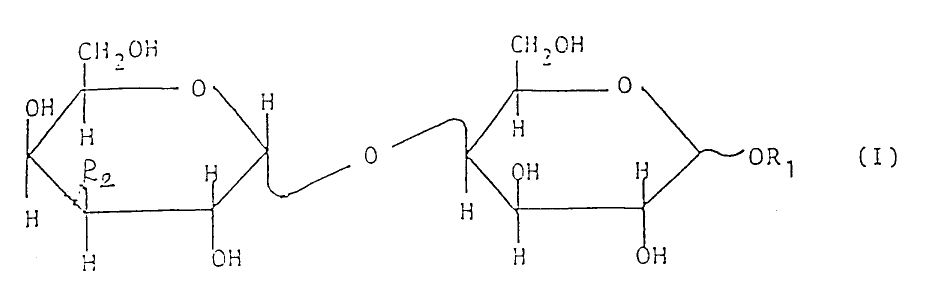 wherein R₁ is hydrogen or an organic residue, provided it does not negatively affect the conditions in connection with the use of the invention and R₂ is
β-D-Galp-(1-3)-β-D-GalNAcp-(1-3)-,
α-D-Galp-(1-3)-, or
β-D-GalNAcp-(1-3)-.

2. A composition according to claim 1, characterized thereby that OR₁ in formula I is in β-configuration.

3. A composition according to claim 1 or 2, characterized thereby that R₁ is a carbohydrate residue.

4. A composition according to claim 1 or 2, characterized thereby that R₁ is alkyl having 1 to 6 carbon atoms.

5. A composition according to any preceding claim, characterized thereby that compound I is present in combination with a pharmaceutically acceptable carrier.

6. A method for determining the presence of the toxine of Shigella Dysenteriae in a sample taken from a mammal including man, comprising determining the degree of interaction between the toxine of the sample and the compound of any of claims 1-4.

## Patentansprüche

1. Zusammensetzung für therapeutische oder diagnostische Verwendung aufgrund ihrer Fähigkeit, das Toxin von Shigella dysenteriae zu binden, **dadurch gekennzeichnet**, daß sie als einen aktiven Bestandteil eine Verbindung der Formel 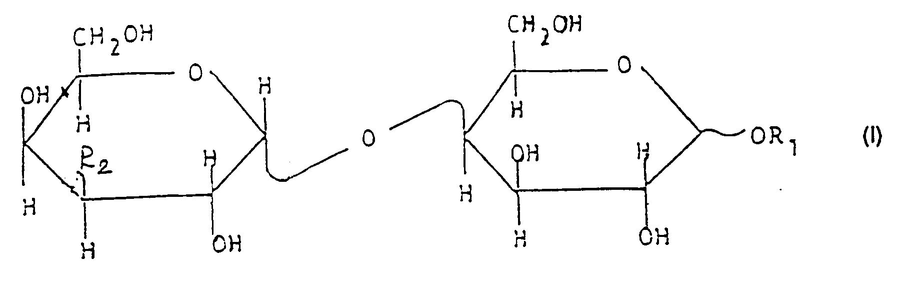 enthält, oder daraus besteht, worin R₁ ein Wasserstoffatom oder einen organischen Rest bedeutet, vorausgesetzt, daß er die Bedingungen in Verbindung mit der Verwendung der Erfindung nicht negativ beeinflußt, und R₂ β-D-Galp-(1-3)-β-D-Gal-NAcp-(1-3)-, α-D-Galp-(1-3) oder β-D-GalNAcp-(1-3)- ist.

2. Zusammensetzung nach Anspruch 1, **dadurch gekennzeichnet**, daß OR₁ in der Formel I in β-Konfiguration ist.

3. Zusammensetzung nach Anspruch 1 oder 2, **dadurch gekennzeichnet**, daß R₁ ein Kohlenhydratrest ist.

4. Zusammensetzung nach Anspruch 1 oder 2, **dadurch gekennzeichnet**, daß R₁ Alkyl mit 1 bis 6 Kohlenstoffatomen ist.

5. Zusammensetzung nach einem der vorausgehenden Ansprüche, **dadurch gekennzeichnet**, daß die Verbindung I in Kombination mit einem pharmazeutisch verträglichen Träger vorliegt.

6. Verfahren zur Bestimmung der Anwesenheit des Toxins von Shigella dysenteriae in einer von einem Säugetier einschließlich des Menschen entnommenen Probe, indem man den Grad der Wechselwirkung zwischen dem Toxin der Probe und der Verbindung nach einem der Ansprüche 1 bis 4 bestimmt.

## Revendications

1. Composition pour une utilisation à des fins thérapeutiques ou de diagnostic en raison de son pouvoir à se lier à la toxine de Shigella Dysenteriae, caractérisée en ce qu'elle contient ou qu'elle se compose de , en tant que constituant actif , un composé de formule (I) 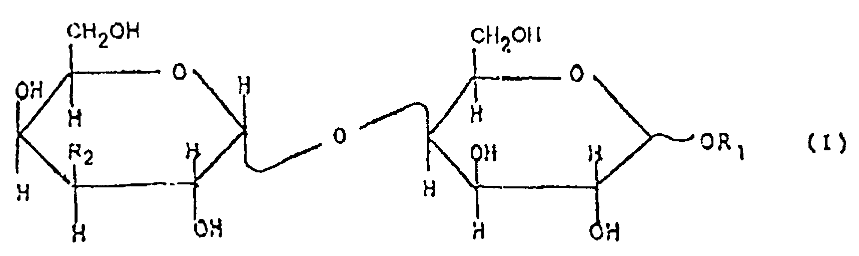 dans laquelle R₁ représente un atome d'hydrogène ou un résidu organique , à condition qu'il n'affecte pas de façon négative les conditions à propos de l'utilisation de l'invention et R₂ représente
β-D-Galp-(1-3)-β-D-GalNAcp-(1-3)-,
α-D-Galp-(1-3)-, ou
β-D-GalNAcp-(1-3)-.

2. Composition selon la revendication 1, caractérisée en ce que OR₁ dans la formule I est dans une configuration β.

3. Composition selon la revendication 1 ou 2, caractérisée en ce que R₁ représente un résidu carbohydrate.

4. Composition selon la revendication 1 ou 2, caractérisée en ce que R₁ représente un groupement alkyle ayant 1 à 6 atomes de carbone.

5. Composition selon l'une quelconque des revendications précédentes, caractérisée en ce que le composé I est présent en combinaison avec un porteur acceptable pharmaceutiquement.

6. Procédé pour déterminer la présence de la toxine de Shigella Dysenteriae dans un échantillon prélevé d'un mammifère, y compris l'homme, comprenant la détermination du degré d'interaction entre la toxine de l'échantillon et le composé de l'une quelconque des revendications 1 à 4.
